# EUROPÄISCHE PATENTANMELDUNG

(11) **EP 1 195 150 A1**
(43) Veröffentlichungstag der Anmeldung: **10.04.2002**
(21) Anmeldenummer: 01122342.7
(22) Anmeldetag: 19.09.2001
(51) Int. Cl.: A61F 2/42

(54) **Fingergrundgelenksimplantat**

(30) Priorität: 22.09.2000 DE 10047033; 07.09.2001 DE 10143865
(71) Anmelder: Ceramtec AG Innovative Ceramic Engineering, 73207 Plochingen (DE); Universität Heidelberg, 69117 Heidelberg (DE)
(72) Erfinder: Boumann, Hans-Werner, Dr., 69121 Heidelberg (DE); Paff, Hans-Georg, 73760 Ostfildern (DE); Sennwald, Gontran, Dr., 3074 Muri b. Bern (CH); Schwarz, Markus, Dr., 82067 Ebenhausen (DE)
(74) Vertreter: Uppena, Franz, Dr.

(57) **Zusammenfassung**

Der Ersatz eines Fingergrundgelenks erfolgt nach dem Stand der Technik beispielsweise durch Interposition von Weichteilgewebe oder eines Abstandshalters, der aber keinen Gelenkersatz darstellt. Bei Gelenkprothesen mit Metall-Metall-Gleitpaarung, insbesondere aber mit Metall-Polyethylen-Gleitpaarung, kann es durch den Abrieb des Werkstoffs zu Osteolysen an den Knochenenden kommen. Dadurch, dass die aus dem Stand der Technik bekannten Metall- oder Kunststoffgelenkprothesen in der Regel mehrteilig sind, kann es zur Lockerung der einzelnen Komponenten kommen, was die Lebensdauer des Implantats beeinträchtigt.

Erfindungsgemäß wird deshalb vorgeschlagen, dass das Implantat (1) ungekoppelt und zweiteilig ist.

## Beschreibung

Die Erfindung betrifft ein Fingergrundgelenksimplantat.

Der Ersatz eines Fingergrundgelenks erfolgt nach dem Stand der Technik beispielsweise durch Interposition von Weichteilgewebe oder eines Abstandshalters aus einem Kunststoff, beispielsweise aus Silastik® . Am häufigsten wird ein Silikon-Spacer verwendet, der nur als Abstandshalter dient und keinen Gelenkersatz darstellt. Nach dessen Einsatz ist das Gelenk instabil und nur sehr eingeschränkt beweglich. Es kann zum vorzeitigen Abrieb des Werkstoffs und dadurch zu Osteolysen an den Knochenenden kommen. Bei Gelenkprothesen mit Metall-Metall-Gleitpaarung, insbesondere aber mit Metall-Polyethylen-Gleitpaarung, kann es durch den Abrieb des Werkstoffs zu Osteolysen an den Knochenenden kommen. Dadurch, dass die aus dem Stand der Technik bekannten Metall- oder Kunststoffgelenkprothesen in der Regel mehrteilig sind, kann es zur Lockerung der einzelnen Komponenten kommen, was die Lebensdauer des Implantats beeinträchtigt. Da die Fingergrundgelenksimplantate, die Fingergelenksendoprothesen, in der Regel gekoppelt sind, führt das häufig zur vorzeitigen Auslockerung des Gelenks.

Aufgabe der vorliegenden Erfindung ist es, ein künstliches Fingergrundgelenk zu schaffen, das bei einer verschleißfreien Gleitpaarung eine anatomische Bewegung ermöglicht. Darüber hinaus sollen biologisch innerte Werkstoffe einen langzeitigen Gelenkersatz ermöglichen.

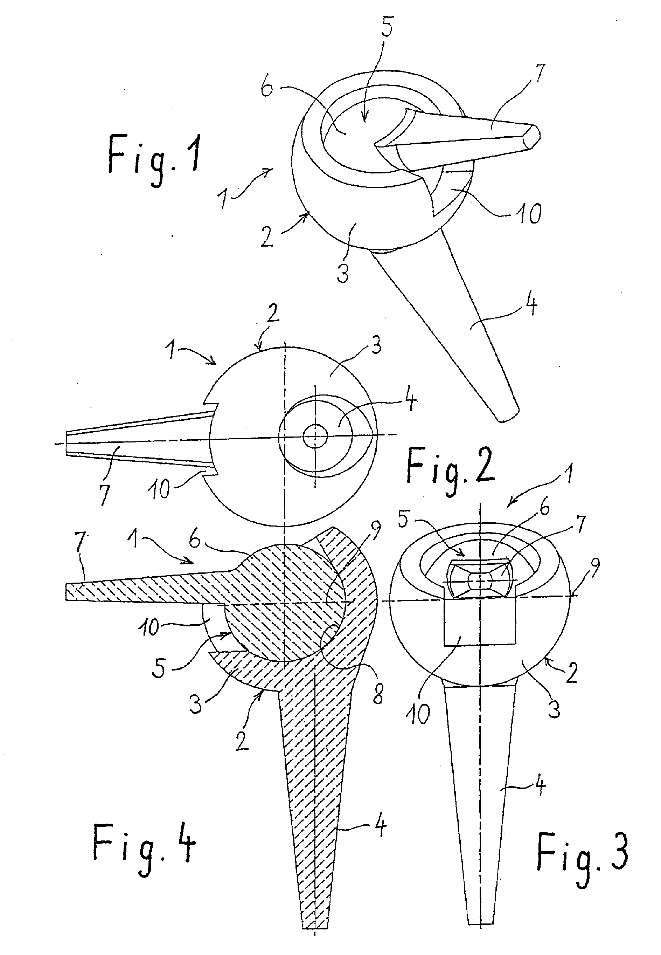

Die Aufgabe wird gelöst durch ein ungekoppeltes, zweiteiliges Implantat mit kongruenten, sphärischen Gleitflächen. Es ist in den Figuren 1 bis 4 dargestellt. Die Figur 1 zeigt eine perspektivische Ansicht. Das Fingergrundgelenksimplantat 1 besteht aus zwei monolithischen Komponenten, der proximalen Komponente 2, bestehend aus dem hohlkugelförmigen Pfannenlager 3 mit dem proximalen Schaft 4, sowie der distalen Komponente 5, bestehend aus einer in dem Pfannenlager 3 gelagerten Kugel 6, die mittels des distalen Schafts 7 an den Fingerknochen implantiert ist.

Die Figur 2 zeigt die proximale Ansicht. Die Figur 3 zeigt eine Ansicht auf das sich in Beugestellung befindliche Implantat und die Figur 4 einen Schnitt durch das Implantat in der Stellung nach Figur 3 in der Seitenansicht.

Wie aus der Figur 4 ersichtlich ist, reicht die Lagerfläche 8 des Pfannenlagers 3 über die Äquatorialebene 9 hinaus und bietet dadurch einen großen Luxationsschutz, einen Schutz vor Verrenkungen des Fingergliedes. Bei vollständiger Streckung der Fingerglieder ist eine Abduktion/Adduktion von bis zu +/- 30 Winkelgraden möglich. Die Adduktion wird durch eine bewegungsgerechte Aussparung 10 in der proximalen Komponente 2 gewährleistet. Bei zunehmender Flexion, das heißt bei zunehmender Beugung des Fingers, wird das Implantat so geführt, dass sowohl die Abduktion als auch die Adduktion zunehmend eingeschränkt wird.

Die Vorteile des erfindungsgemäßen Implantats liegen darin, dass es vollständig aus Keramik, vorzugsweise aus Aluminiumoxid-Keramik besteht. Sowohl die proximalen als auch die distalen Implantationskomponenten sind jeweils monolithisch. Durch die Form der Prothese wird eine gute Beweglichkeit bei anatomischer seitlicher Führung ermöglicht. Die Form der Lagerung sowie das Material selbst gewährleisten eine hohen Abriebfestigkeit und damit eine langjährige Haltbarkeit. Die Implantation erfolgt zementfrei. Dazu weisen der proximale Schaft 4 und der distale Schaft 7 eine das Knocheneinwachsen, die Osteointegration, fördernde Beschichtung auf, beispielsweise Hydroxylapatit. Die Schäfte können auch eine Struktur aufweisen, die porös ist und damit das Einwachsen des Knochengewebes begünstigt.

Aufgrund ihrer Form ist die erfindungsgemäße Prothese besonders zum Ersatz zerstörter und instabiler Fingergrundgelenke, insbesondere bei Rheumatikern, geeignet.

## Patentansprüche

1. Fingergrundgelenksimplantat, **dadurch gekennzeichnet, dass** das Implantat (1) ungekoppelt und zweiteilig ist.

2. Fingergrundgelenksimplantat nach Anspruch 1, **dadurch gekennzeichnet, dass** das Implantat (1) aus zwei monolithischen Komponenten zusammengesetzt ist, der proximalen Komponente (2), bestehend aus dem hohlkugelförmigen Pfannenlager (3) mit dem proximalen Schaft (4), sowie der distalen Komponente (5), bestehend aus einer in dem Pfannenlager (3) gelagerten Kugel (6), die mittels des distalen Schafts (7) an den Fingerknochen implantiert ist.

3. Fingergrundgelenksimplantat nach Anspruch 1 oder 2, **dadurch gekennzeichnet, dass** das Implantat (1) kongruente, sphärische Gleitflächen aufweist, von denen die eine das hohlkugelförmige Pfannenlager (3) und die andere die Oberfläche der Kugel (6) ist.

4. Fingergrundgelenksimplantat nach einem der Ansprüche 1 bis 3, **dadurch gekennzeichnet, dass** die Lagerfläche (8) des Pfannenlagers (3) als Luxationsschutz über die Äquatorialebene (9) hinausreicht.

5. Fingergrundgelenksimplantat nach einem der Ansprüche 1 bis 4, **dadurch gekennzeichnet, dass** eine Adduktion durch eine bewegungsgerechte Aussparung (10) in der proximalen Komponente (2) gewährleistet ist und bei vollständiger Streckung der Fingerglieder eine Abduktion/Adduktion von bis zu +/- 30 Winkelgraden möglich ist.

6. Fingergrundgelenksimplantat nach einem der Ansprüche 1 bis 5, **dadurch gekennzeichnet, dass** bei zunehmender Flexion, das heißt bei zunehmender Beugung des Fingers, die Führung des distalen Schaftes (7) so gestaltet ist, dass sowohl die Abduktion als auch die Adduktion zunehmend eingeschränkt ist.

7. Fingergrundgelenksimplantat nach einem der Ansprüche 1 bis 6, **dadurch gekennzeichnet, dass** es vollständig aus Keramik besteht.

8. Fingergrundgelenksimplantat nach Anspruch 7, **dadurch gekennzeichnet, dass** es vollständig aus Aluminiumoxid-Keramik besteht.

9. Fingergrundgelenksimplantat nach einem der Ansprüche 1 bis 8, **dadurch gekennzeichnet, dass** der proximale Schaft (4) und der distale Schaft (7) eine das Knocheneinwachsen, die Osteointegration, fördernde Beschichtung aufweisen.

10. Fingergrundgelenksimplantat nach Anspruch 9, **dadurch gekennzeichnet, dass** die das Knocheneinwachsen fördernde Beschichtung Hydroxylapatit ist.

11. Fingergrundgelenksimplantat nach einem der Ansprüche 1 bis 8, **dadurch gekennzeichnet, dass** der proximale Schaft (4) und der distale Schaft (7) eine das Knocheneinwachsen, die Osteointegration, fördernde poröse Struktur aufweisen.
